# EUROPEAN PATENT SPECIFICATION

(11) **EP 1 007 656 B1**
(45) Date of publication and mention of the grant of the patent: **13.09.2006**
(21) Application number: 98907490.1
(22) Date of filing: 12.02.1998
(51) Int. Cl.: C12N 15/11, A61K 31/70, C07H 21/04

(54) **MODIFIED PROTEIN KINASE A-SPECIFIC HYBRID OLIGONUCLEOTIDE IN COMBINATION WITH PACLITAXOL AND METHODS OF THEIR USE**
VERÄNDERTES PROTEIN KINASE A-SPEZIFISCHES HYBRID OLIGONUKLEOTID IN KOMBINATION MIT PACLITAXOL UND VERFAHREN IHRER ANWENDUNG
OLIGONUCLEOTIDE HYBRIDE MODIFIE SPECIFIQUE DE LA PROTEINE KINASE A EN COMBINAISON AVEC LE PACLITAXOL ET LEUR METHODES D'UTILISATION

(30) Priority: 12.03.1997 US 40740 P
(43) Date of publication of application: 14.06.2000
(73) Proprietor: IDERA PHARMACEUTICALS, INC., Cambridge, MA 02139 (US)
(72) Inventor: AGRAWAL, Sudhir, Shrewsbury, MA 01545 (US)
(74) Representative: Grund, Martin
(86) International application number: PCT/US1998/003003
(87) International publication number: WO 1998/040479

(56) References cited:
- EP-A- 0 490 077
- WO-A-94/23028
- WO-A-96/16976
- WO-A-96/31600
- WO-A-97/11171
- US-A- 2003 105 035
- LU, Z. ET AL.: "In vivo stability, pharmacokinetics, and metabolism of a ' hybrid ' oligonucleotide phosphorothioate in rats." PROC ANNU MEET AM ASSOC CANCER RES, (MARCH 1995). 36, PAGE 411. ABSTRACT 2450., XP002025691 & EIGHTY-SIXTH ANNUAL MEETING OF THE AMERICAN ASSOCIATION FOR CANCER RESEARCH, TORONTO, ONTARIO, CANADA, MARCH 18-22, 1995,
- ZHANG, R. ET AL.: "In vivo stability, disposition and metabolism of a " hybrid " oligonucleotide phosphorothioate in rats." BIOCHEMICAL PHARMACOLOGY, (1995 AUG 8) 50 (4) 545-56., XP000644798
- GALBRAITH, W. ET AL.: "Complement activation and hemodynamic changes following intravenous administration of phosphorothioate oligonucleotides in the monkey" ANTISENSE RESEARCH AND DEVELOPMENT, vol. 4, 1994, US, pages 201-206, XP002025692 cited in the application
- NESTEROVA, M. & CHO-CHUNG, Y.: "A single injection protein kinase A-directed antisense treatment to inihibit tumour growth" NATURE MEDICINE, vol. 1, June 1995, pages 528-533, XP002025693 cited in the application
- MONIA, B. ET AL.: "EVALUATION OF 2'-MODIFIED OLIGONUCLEOTIDES CONTAINING 2'-DEOXY GAPS AS ANTISENSE INHIBITORS OF GENE EXPRESSION" JOURNAL OF BIOLOGICAL CHEMISTRY, vol. 268, no. 19, 5 July 1993, pages 14514-14522, XP000576145
- PISETSKY, D. & REICH, C.: "STIMULATION OF IN VITRO PROLIFERATION OF MURINE LYMPHOCYTES BY SYNTHETIC OLIGODEOXYNULEOTIDES" MOLECULAR BIOLOGY REPORT, vol. 18, no. 3, October 1993, pages 217-221, XP000610055
- ZHAO, Q. ET AL.: "EFFECT OF DIFFERENT CHEMICALLY MODIFIED OLIGODEOXYNUCLEOTIDES ON IMMUNE STIMULATION" BIOCHEMICAL PHARMACOLOGY, vol. 51, no. 2, 26 January 1996, pages 173-182, XP000610208
- YU, D. ET AL.: "Hybrid oligonucleotides: Synthesis, biophysical properties, stability studies, and biological activity." BIOORGANIC AND MEDICINAL CHEMISTRY, (1996) 4/10 (1685-1692)., XP000644792

## Description

### BACKGROUND OF THE INVENTION

The development of effective cancer therapies has been a major focus of biomedical research. Surgical procedures have been developed and used to treat patients whose tumors are confined to particular anatomical sites. However, at presentation, only about 25% of patients have tumors that are truly confined and amenable to surgical treatment alone (Slapak et al. in Harrison's Principles of Internal Medicine (Isselbacher et al., eds.) McGraw-Hill, Inc., NY (1994) pp. 1826-1850). Radiation therapy, like surgery, is a local modality whose usefulness in the treatment of cancer depends to a large extent on the inherent radiosensitivity of the tumor and its adjacent normal tissues. However, radiation therapy is associated with both acute toxicity and long term sequelae. Furthermore, radiation therapy is known to be mutagenic, carcinogenic, and teratogenic (Slapak et al., ibid.).

Systemic chemotherapy alone or in combination with surgery and/or radiation therapy is currently the primary treatment available for disseminated malignancies. However, conventional chemotherapeutic agents which either block enzymatic pathways or randomly interact with DNA irrespective of the cell phenotype, lack specificity for killing neoplastic cells. Thus, systemic toxicity often results from standard cytotoxic chemotherapy. More recently, the development of agents that block replication, transcription, or translation in transformed cells, and at the same time defeat the ability of cells to become resistant, has been the goal of many approaches to chemotherapy.

One strategy is to down regulate the expression of a gene associated with the neoplastic phenotype in a cell. A technique for turning off a single activated gene is the use of antisense oligodeoxynucleotides and their analogues for inhibition of gene expression (Zamecnik et al. (1978) *Proc. Natl. Acad. Sci. (USA)* **75**:280-284). An antisense oligonucleotide targeted at a gene involved in the neoplastic cell growth should specifically interfere only with the expression of that gene, resulting in arrest of cancer cell growth. The ability to specifically block or down-regulate expression of such genes provides a powerful tool to explore the molecular basis of normal growth regulation, as well as the opportunity for therapeutic intervention (see, e.g., Cho-Chung (1993) *Curr. Opin. Thera. Patents* **3** :1737-1750). The identification of genes that confer a growth advantage to neoplastic cells as well as other genes causally related to cancer and the understanding of the genetic mechanism(s) responsible for their activation makes the antisense approach to cancer treatment possible.

One such gene encodes the RI_{α} subunit of cyclic AMP (cAMP)-dependent protein kinase A (PKA) (Krebs (1972) *Curr. Topics Cell. Regul.* **5**:99-133) . Protein kinase is bound by cAMP, which is thought to have a role in the control of cell proliferation and differentiation (see, e.g., Cho-Chung (1980) *J. Cyclic Nucleotide Res.* **6**:163-167). There are two types of PKA, type I (PKA-I) and type II (PKA-II), both of which share a common C subunit but each containing distinct R subunits, RI and RII, respectively (Beebe et al. in *The Enzymes: Control by Phosphorylation,* 17 (A) : 43-111 (Academic, New York, 1986). The R subunit isoforms differ in tissue distribution (Øyen et al. (1988) *FEBS Lett.* **229** :391-394 ; Clegg et al. (1988) *Proc. Natl. Acad. Sci. (USA)* 85:3703-3707) and in biochemical properties (Beebe et al. in *The Enzymes: Control by Phosphorylation,* 17 (A): :43 -111 (Academic Press, NY, 1986) ; Cadd et al. (1990) *J. Biol. Chem.* 265:19502-19506). The two general isoforms of the R subunit also differ in their subcellular localization: RI is found throughout the cytoplasm; whereas RI localizes to nuclei, nucleoli, Golgi apparatus and the microtubule-organizing center (see, e.g., Lohmann in *Advances in Cyclic Nucleotide and Protein Phosphorylation Research,* **18**:63-117 (Raven, New York, 1984; and Nigg et al. (1985) *Cell* 41:1039-1051).

An increase in the level of RI_{α} expression has been demonstrated in human cancer cell lines and in primary tumors, as compared with normal counterparts, in cells after transformation with the Ki*-ras* oncogene or transforming growth factor-α, and upon stimulation of cell growth with granulocyte-macrophage colony-stimulating factor (GM-CSF) or phorbol esters (Lohmann in *Advances in Cyclic Nucleotide and Protein Phosphorylation Research,* **18**:63-117 (Raven, New York, 1984); and Cho-Chung (1990) *Cancer Res.* 50 : 7093-7100 ). Conversely, a decrease in the expression of RI_{α} has been correlated with growth inhibition induced by site-selective CAMP analogs in a broad spectrum of human cancer cell lines (Cho-Chung (1990) *Cancer Res.* 50:7093-7100). It has also been determined that the expression of RI/PKA-I and RII/PKA-II has an inverse relationship during ontogenic development and cell differentiation (Lohmann in *Advances in Cyclic Nucleotide and Protein Phosphorylation Research,* Vol. 18 , 63-117 (Raven, New York, 1984); Cho-Chung (1990) *Cancer Res.* 50:7093-7100). The RI_{α} subunit of PKA has thus been hypothesized to be an ontogenic growth-inducing protein whose constitutive expression disrupts normal ontogenic processes, resulting in a pathogenic outgrowth, such as malignancy (Nesterova et al . (1995) *Nature Medicine* **1**: 528-533).

Antisense oligonucleotides directed to the RI_{α} gene have been prepared. U.S. Patent No. 5,271,941 describes phosphodiester-linked antisense oligonucleotides complementary to a region of the first 100 N-terminal amino acids of RI_{α} which inhibit the expression of RI_{α} in leukemia cells *in vitro.* In addition, antisense phosphorothioate oligodeoxynucleotides corresponding to the N-terminal 8-13 codons of the RI_{α} gene was found to reduced *in vivo* tumor growth in nude mice (Nesterova et al. (1995) *Nature Med.* **1**:528-533).

Unfortunately, problems have been encountered with the use of phosphodiester-linked (PO) oligonucleotides and some phosphorothioate-linked (PS) oligonucleotides. It is known that nucleases in the serum readily degrade PO oligonucleotides. Replacement of the phosphodiester internucleotide linkages with phosphorothioate internucleotide linkages has been shown to stabilize oligonucleotides in cells, cell extracts, serum, and other nuclease-containing solutions (see, e.g., Bacon et al. (1990) *Biochem. Biophys. Meth.* **20**:259) as well as *in vivo* (Iversen (1993) *Antisense Research and Application* (Crooke, ed) CRC Pres s , 461). However, some PS oligonucleotides have been found to exhibit an immunostimulatory response, which in certain cases may be undesirable. For example, Galbraith et al. *(Antisense Res. & Dev.* (1994) **4**:201-206) disclose complement activation by some PS oligonucleotides. Henry et al. *(Pharm. Res.* (1994) **11**: PPDM8082) disclose that some PS oligonucleotides may potentially interfere with blood clotting.

WO 96/16976 concerns an antisense oligonucleotide and carcinostatic agent containing the same. EP-A-0 490 077 describes antisense oligonucleotides for the treatment of cancer. WO 94/23028 states that modified oligonucleotides have improved anti-influenza activity. Lu, Z, et al. discuss the "in vivo stability, pharmacokinetics, and metabolism of a hybrid oligonucleotide phosphorothioare in rats" (Proc. Annu. Meet. Am. Assoc. Cancer Res., 36, page 411, abstract 2450). Zhang R. et aL describe the "in vivo stability, disposition and metabolism of a "hybrid" oligonucleotide phosphorothioate in rats" (Biochem Pharmacol 1995 Aug 8;50(4):545-56). Nesterova, M. et al state that a single injection protein kinase A-directed antisense treatment can inhibit tumour growth (Nature Medicine, vol. 1, 1995, pages 528-533). Monia, B. et al. evaluate 2'-modified oligonucleotides containing 2'-deoxy gaps as antisense inhibitors of gene expression (Journal of Biological Chemistry, vol. 268, no. 19, 1993, pages 14514-14522). Zhao, Q. et al. report on the effect of different chemically modified oligodeoxynucleotides on immune stimulation (Biochemical Pharmacology, voL 51, no. 2, 1996, pages 173-182). WO 96/31600 discloses a method of modulating gene expression without depleting complement. Yu, D. et aL report on hybrid oligonucleotides, their synthesis, biophysical properties, stability studies, and biological activity (Bioorganic and Medicinal Chemistry, 1996, 4/10, pages 1685-1692). WO 97/11171 describes modified protein kinase A-specific oligonucleotides and methods of their use.

There is, therefore, a need for modified oligonucleotides directed to cancer-related genes that retain gene expression inhibition properties while producing fewer side effects than conventional oligonucleotides.

### SUMMARY OF THE INVENTION

The present invention relates to the use of modified oligonucleotides as claimed in claim 1. Modified oligonucleotides that down-regulate the expression of the RI_{α} gene while producing fewer side effects than conventional oligonucleotides are disclosed. In particular, modified oligonucleotides that demonstrate reduced mitogenicity, reduced activation of complement and reduced antithrombotic properties, relative to conventional oligonucleotides are disclosed.

It is also known that some PS oligonucleotides cause an immunostimulatory response in subjects to whom they have been administered, which may be undesirable in some cases.

It is known that exclusively phosphodiester- or exclusively phosphorothioate-linked oligonucleotides directed to the first 100 nucleotides of the RI_{α} nucleic acid inhibit cell proliferation.

It has now been discovered that modified oligonucleotides complementary to the protein kinase A RI_{α} subunit gene inhibit the growth of tumors *in vivo.* With at least the activity of a comparable PO- or PS-linked oligonucleotide with fewer side effects.

This finding has been exploited to produce the present disclosure which in a first aspect, includes synthetic hybrid, inverted hybrid, and inverted chimeric oligonucleotides and compositions of matter for specifically down-regulating protein kinase A subunit RI_{α} gene expression with reduced side effects. Such inhibition of gene expression is useful as an alternative to mutant analysis for determining the biological function and role of protein kinase A-related genes in cell proliferation and tumor growth. Such inhibition of RI_{α} gene expression can also be used to therapeutically treat diseases and disorders that are caused by the over-expression or inappropriate expression of the gene.

As used herein, the term "synthetic oligonucleotide" includes chemically synthesized polymers of three up to 50, preferably from about 15 to about 30, and most preferably, 18 ribonucleotide and/or deoxyribonucleotide monomers connected together or linked by at least one, and preferably more than one, 5' to 3' internucleotide linkage.

For purposes of the invention, the term "oligonucleotide sequence that is complementary to a genomic region or an RNA molecule transcribed therefrom" is intended to mean an oligonucleotide that binds to the nucleic acid sequence under physiological conditions, e.g., by Watson-Crick base pairing (interaction between oligonucleotide and single-stranded nucleic acid) or by Hoogsteen base pairing (interaction between oligonucleotide and double-stranded nucleic acid) or by any other means including in the case of a oligonucleotide binding to RNA, causing pseudoknot formation. Binding by Watson-Crick or Hoogsteen base pairing under physiological conditions is measured as a practical matter by observing interference with the function of the nucleic acid sequence.

In one preferred embodiment according to this aspect of the invention, the oligonucleotide is a core region hybrid oligonucleotide comprising a region of at least two deoxyribonucleotides, flanked by 5' and 3' ribonucleotide regions, each having at least four ribonucleotides. A hybrid oligonucleotide having the sequence set forth in the Sequence Listing as SEQ ID NO:4 is one particular embodiment. In some embodiments, each of the 3' and 5' flanking ribonucleotide regions of an oligonucleotide of the invention comprises at least four contiguous, 2'-O-substituted ribonucleotides.

For purposes of the invention, the term "2'-O-substituted" means substitution of the 2' position of the pentose moiety with an -O- lower alkyl group containing 1-6 saturated or unsaturated carbon atoms, or with an -O-aryl or allyl group having 2-6 carbon atoms, wherein such alkyl, aryl or allyl group may be unsubstituted or may be substituted, e.g., with halo, hydroxy, trifluoromethyl, cyano, nitro, acyl, acyloxy, alkoxy, carboxyl, carbalkoxyl, or amino groups; or with a hydroxy, an amino or a halo group, but not with a 2'-H group.

In some embodiments, each of the 3' and 5' flanking ribonucleotide regions of an oligonucleotide of the invention comprises at least one 2'-O-alkyl substituted ribonucleotide. In one preferred embodiment, the 2'-O-alkylsubstituted nucleotide is a 2'-O-methyl ribonucleotide. In other preferred embodiments, the 3' and 5' flanking ribonucleotide regions of an oligonucleotide of the invention comprises at least four 2'-O-methyl ribonucleotides. In preferred embodiments, the ribonucleotides and deoxyribonucleotides of the hybrid oligonucleotide are linked by phosphorothioate internucleotide linkages. In particular embodiments, this phosphorothioate region or regions have from about four to about 18 nucleosides joined to each other by 5' to 3' phosphorothioate linkages, and preferably from about 5 to about 18 such phosphorothioate-linked nucleosides. The phosphorothioate linkages may be mixed Rₚ and Sₚ enantiomers, or they may be stereoregular or substantially stereoregular in either Rₚ or Sₚ form (see Iyer et al. (1995) *Tetrahedron Asymmetry* 6:1051-1054).

An inverted hybrid oligonucleotide comprising a region of at least four ribonucleotides flanked by 3' and 5' deoxyribonucleotide regions of at least two deoxyribonucleotides is also disclosed. The structure of this oligonucleotide is "inverted" relative to traditional hybrid oligonucleotides. In some embodiments, the 2'-O-substituted RNA region has from about four to about ten 2'-O-substituted nucleosides joined to each other by 5' to 3' internucleoside linkages, and most preferably from about four to about six such 2'-O-substituted nucleosides. Some oligonucleotides have a ribonucleotide region comprising at least five contiguous ribonucleotides. The overall size of the inverted hybrid oligonucleotide can be 18. The 2'-O-substituted ribonucleosides can be linked to each other through a 5' to 3' phosphorothioate, phosphorodithioate, phosphotriester, or phosphodiester linkages. The phosphorothioate 3' or 5' flanking region (or regions) has from about four to about 18 nucleosides joined to each other by 5' to 3' phosphorothioate linkages, and can be from about 5 to about 18 such phosphorothioate-linked nucleosides. The phosphorothioate regions can have at least 5 phosphorothioate-linked nucleosides. An oligonucleotide having substantially the nucleotide sequence set forth in the Sequence Listing as SEQ ID NO: 6 is also disclosed. The ribonucleotide region can comprise 2'-O-substituted ribonucleotides, such as 2'-O-alkyl substituted ribonucleotides. A hybrid oligonucleotide whose ribonucleotide region comprise at least one 2'-O-methyl ribonucleotide is also disclosed.

All of the nucleotides in the inverted hybrid oligonucleotide can be linked by phosphorothioate internucleotide linkages. The deoxyribonucleotide flanking region or regions can have from about four to about 18 nucleosides joined to each other by 5' to 3' phosphorothioate linkages, and preferably from about 5 to about 18 such phosphorothioate-linked nucleosides. The deoxyribonucleotide 3' and 5' flanking regions of the hybrid oligonucleotides of the invention can have about 5 phosphorothioate-linked nucleosides. The phosphorothioate linkages may be mixed Rₚ and Sₚ enantiomers, or they may be stereoregular or substantially stereoregular in either Rₚ or Sₚ form (see lyer et al. (1995) *Tetrahedron Asymmetry* 6:1051-1054).

A composition of matter for inhibiting the expression of protein kinase A subunit RI_{α} with reduced side effects is also disclosed, the composition comprising an inverted hybrid oligonucleotide.

An inverted chimeric oligonucleotide comprising an oligonucleotide nonionic region of at least four nucleotides flanked by one or more, and preferably two oligonucleotide phosphorothioate regions is also disclosed. Such a chimeric oligonucleotide has a structure that is "inverted" relative to traditional chimeric oligonucleotides. An inverted chimeric oligonucleotide of the invention may have substantially the nucleotide sequence set forth in the Sequence Listing as SEQ ID NO:1. The oligonucleotide nonionic region may comprise about four to about 12 nucleotides joined to each other by 5' to 3' nonionic linkages. The nonionic region contains alkylphosphonate and/or phosphoramidate and/or phosphotriester internucleoside linkages. The oligonucleotide nonionic region may comprise six nucleotides. The oligonucleotide may have a nonionic region having from about six to about eight methylphosphonate-linked nucleosides, flanked on either side by phosphorothioate regions, each having from about six to about ten phosphorothioate-linked nucleosides. The flanking region or regions may be phosphorothioate nucleotides. The flanking region or regions may have from about four to about 24 nucleosides joined to each other by 5' to 3' phosphorothioate linkages, and preferably from about six to about 16 such phosphorothioate-linked nucleosides. The phosphorothioate regions may have from about five to about 15 phosphorothioate-linked nucleosides. The phosphorothioate linkages may be mixed Rₚ and Sₚ enantiomers, or they may be stereoregular or substantially stereoregular in either Rₚ or Sₚ form (see Iyer et al. (1995) *Tetrahedron Asymmetry* 6:1051-2054).

A composition of matter for inhibiting the expression of protein kinase A subunit RI_{α} with reduced side effects is also disclosed, the composition comprising an inverted chimeric oligonucleotide.

A method of inhibiting the proliferation of cancer cells *in vitro* is also disclosed. In this method, an oligonucleotide of the invention is administered to the cells.

A therapeutic composition comprising an oligonucleotide of the invention in a pharmaceutically acceptable carrier is also disclosed.

A method of treating cancer in an afflicted subject with reduced side effects is also disclosed This method comprises administering a therapeutic composition of the invention to the subject in which the protein kinase A subunit RI_{α} gene is being over-expressed.

2'-O-substituted ribonucleotide regions may well include from one to all nonionic internucleoside linkages. Alternatively, nonionic regions may have from one to all 2'-O-substituted ribonucleotides. Moreover, oligonucleotides according to the invention may contain combinations of one or more 2'-O-substituted ribonucleotide region and one or more nonionic region, either or both being flanked by phosphorothioate regions. (See *Nucleosides & Nucleotides* **14**:1031-1035 (1995) for relevant synthetic techniques.

### BRIEF DESCRIPTION OF THE DRAWINGS

The foregoing and other objects of the present invention, the various features thereof, as well as the invention itself may be more fully understood from the following description, when read together with the accompanying drawings in which:

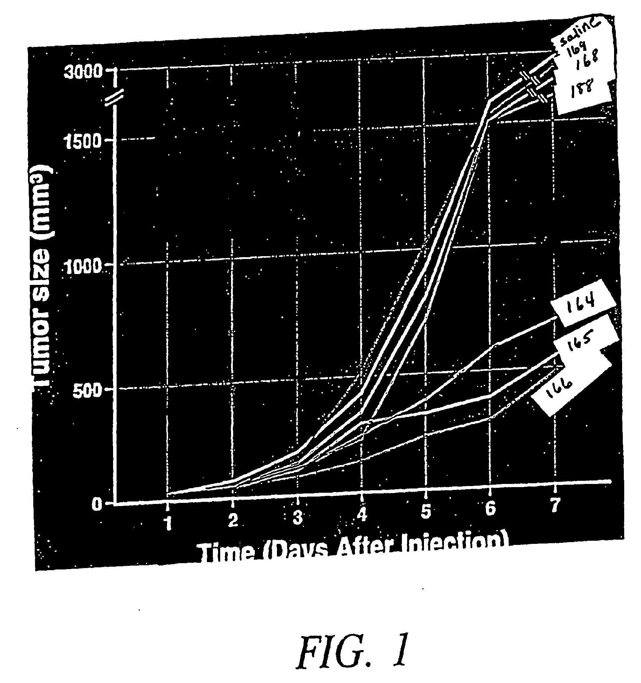

FIG. 1 is a graphic representation showing the effect of modified oligonucleotides of the invention on tumor size in a mouse relative to various controls.

### DESCRIPTION OF THE PREFERRED DISCLOSURES

Synthetic oligonucleotides of the hybrid, inverted hybrid, and inverted chimeric oligonucleotides as described above.

Such synthetic hybrid, inverted hybrid, and inverted chimeric oligonucleotides have a nucleotide sequence complementary to a genomic region or an RNA molecule transcribed therefore encoding the RI_{α} subunit of PKA. These oligonucleotides are about 15 to about 30 nucleotides in length, preferably about 15 to 25 nucleotides in length, but most preferably, are about 18 nucleotides long. The sequence of this gene is known. Thus, an oligonucleotide of the invention can have any nucleotide sequence complementary to any region of the gene. Three non-limiting examples of an 18mer of the disclosure has the sequence set forth below in TABLE 1 as SEQ ID NOS:1, 4, and 6.

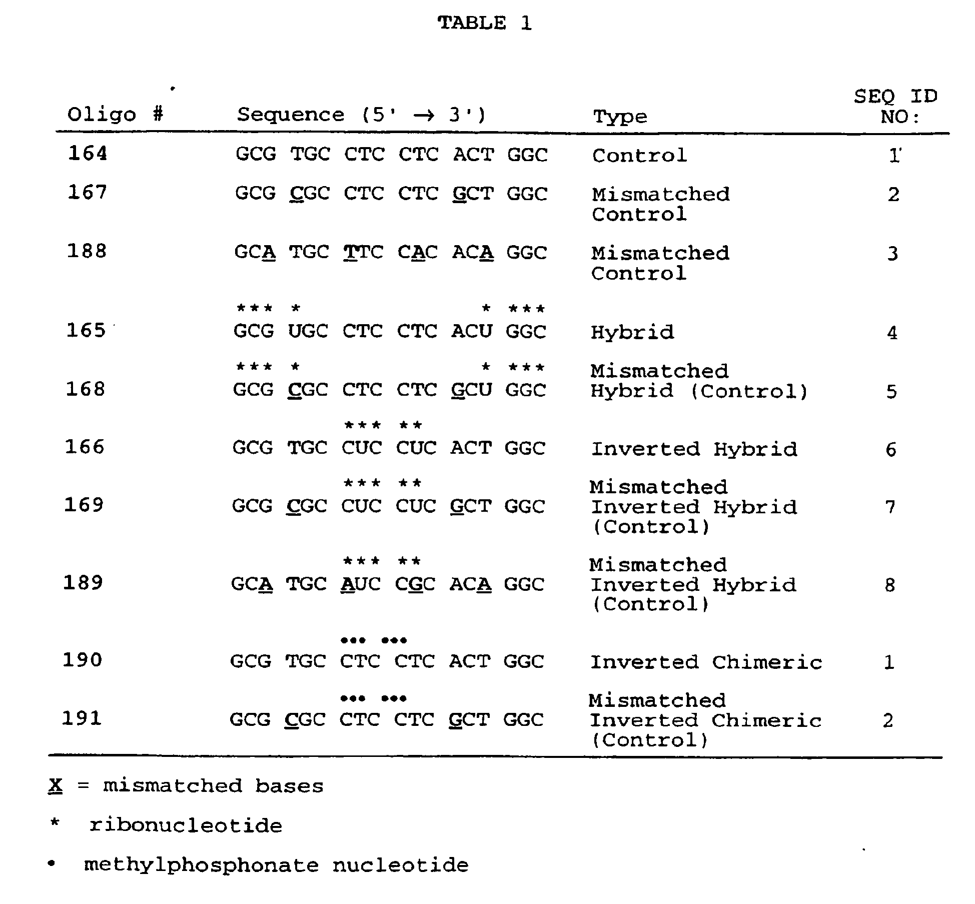

Oligonucleotides having greater than 18 oligonucleotides are also contemplated by the invention. These oligonucleotides have up to 25 additional nucleotides extending from the 3', or 5' terminus, or from both the 3' and 5' termini of, for example, the 18mer with SEQ ID NOS:1, 4, or 6, without diminishing the ability of these oligonucleotides to down regulate RI_{α} gene expression. Alternatively, other oligonucleotides may have fewer nucleotides than, for example, oligonucleotides having SEQ ID NOS:1, 4, or 6. Such shortened oligonucleotides maintain at least the antisense activity of the parent oligonucleotide to down-regulate the expression of the RI_{α} gene, or have greater activity.

The oligonucleotides can be prepared by art recognized methods. Oligonucleotides with phosphorothioate linkages can be prepared manually or by an automated synthesizer and then processed using methods well known in the field such as phosphoramidite (reviewed in Agrawal et al. (1992) *Trends Biotechnol.* **10**: 152-158, *see, e.g.,* Agrawal et al. (1988) *Proc. Natl. Acad. Sci.* (USA) **85** : 7079-7083 ) or H-phosphonate *(see, e.g.,* Froehler (1986) *Tetrahedron Lett.* **27**:5575-5578) chemistry. The synthetic methods described in Bergot et al. *(J.Chromatog.* (1992) **559**:35-42) can also be used. Examples of other chemical groups include alkylphosphonates, phosphorodithioates, alkylphosphonothioates, phosphoramidates, 2'-O-methyls, carbamates, acetamidate, carboxymethyl esters, carbonates, and phosphate triesters. Oligonucleotides with these linkages can be prepared according to known methods (*see, e.g.,* Goodchild (1990) *Bioconjugate Chem.* **2**:165-187; Agrawal et al. *(Proc. Natl. Acad. Sci.* (USA) (1988) **85**:7079-7083); Uhlmann et al. *(Chem. Rev.* (1990) **90**:534-583 ; and Agrawal et al. *(Trends Biotechnol.* (1992) **10**:152-158)).

The hybrid, inverted hybrid, and inverted chimeric oligonucleotides may have other modifications which do not substantially affect their ability to specifically down-regulate RI_{α} gene expression. These modifications include those which are internal or are at the end(s) of the oligonucleotide molecule and include additions to the molecule at the internucleoside phosphate linkages, such as cholesteryl or diamine compounds with varying numbers of carbon residues between the two amino groups, and terminal ribose, deoxyribose and phosphate modifications which cleave, or crosslink to the opposite chains or to associated enzymes or other proteins which bind to the RI_{α} nucleic acid. Examples of such oligonucleotides include those with a modified base and/or sugar such as arabinose instead of ribose, or a 3', 5'-substituted oligonucleotide having a sugar which, at one or both its 3' and 5' positions is attached to a chemical group other than a hydroxyl or phosphate group (at its 3' or 5' position). Other modified oligonucleotides are capped with a nuclease resistance-conferring bulky substituent at their 3' and/or 5' end(s), or have a substitution in one or both nonbridging oxygens per nucleotide. Such modifications can be at some or all of the internucleoside linkages, as well as at either or both ends of the oligonucleotide and/or in the interior of the molecule (reviewed in Agrawal et al. (1992) *Trends Biotechnol.* **10**:152-158).

The disclosure also provides therapeutic compositions suitable for treating undesirable, uncontrolled cell proliferation or cancer comprise at least one oligonucleotide in accordance with the disclosure, capable of specifically down-regulating expression of the RI_{α} gene, and a pharmaceutically acceptable carrier or diluent. It is preferred that an oligonucleotide used in the therapeutic composition of the disclosure be complementary to at least a portion of the RI_{α} genomic region, gene, or RNA transcript thereof.

As used herein, a "pharmaceutically or physiologically acceptable carrier" includes any and all solvents (including but limited to lactose), dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions of the invention is contemplated. Supplementary active ingredients can also be incorporated into the compositions.

Therapeutic compositions suitable for inhibiting cell proliferation *in vitro* or *in vivo* or for treating-cancer in humans comprises about 25 to 75 mg of a lyophilized oligonucleotide(s) having SEQ ID NOS:1, 4, and/or 6 and 20-75 mg lactose, USP, which is reconstituted with sterile normal saline to the therapeutically effective dosages described herein.

The disclosure also provides methods for treating humans suffering from disorders or diseases wherein the RI_{α} gene is incorrectly or over-expressed. Such a disorder or disease that could be treated using this method includes tumor-forming cancers such as, but not limited to, human colon carcinoma, breast carcinoma, gastric carcinoma, and neuroblastoma. In the method of the disclosure, a therapeutically effective amount of a composition is administered to the human. Such methods of treatment are administered in conjunction with other therapeutic agents.

As used herein, the term "therapeutically effective amount" means the total amount of each active component of the pharmaceutical formulation or method that is sufficient to show a meaningful subject or patient benefit, i.e., a reduction in tumor growth or in the expression of proteins which cause or characterize the cancer.

A "therapeutically effective manner" refers to a route, duration, and frequency of administration of the pharmaceutical formulation which ultimately results in meaningful patient benefit, as described above. In some embodiments of the invention, the pharmaceutical formulation is administered via injection, sublingually, rectally, intradermally, orally, or enterally in bolus, continuous, intermittent, or continuous, followed by intermittent regimens.

The therapeutically effective amount of synthetic oligonucleotide in the pharmaceutical composition of the present invention will depend upon the nature and severity of the condition being treated, and on the nature of prior treatments which the patent has undergone. Ultimately, the attending physician will decide the amount of synthetic oligonucleotide with which to treat each individual patient. Initially, the attending physician will administer low doses of the synthetic oligonucleotide and observe the patient's response. Larger doses of synthetic oligonucleotide may be administered until the optimal therapeutic effect is obtained for the patient, and at that point the dosage is not increased further. It is contemplated that the dosages of the pharmaceutical compositions administered in the method of the present invention should contain about 0.1 to 5.0 mg/kg body weight per day, and preferably 0.1 to 2.0 mg/kg body weight per day. When administered systemically, the therapeutic composition is preferably administered at a sufficient dosage to attain a blood level of oligonucleotide from about 0.01 µM to about 10 µM. Preferably, the concentration of oligonucleotide at the site of aberrant gene expression should be from about 0.01 µM to about 10 µM, and most preferably from about 0.05 µM to about 5 µM. However, for localized administration, much lower concentrations than this may be effective, and much higher concentrations may be tolerated.

Administration of pharmaceutical compositions in accordance with invention or to practice the method of the present invention can be carried out in a variety of conventional ways, such as by oral ingestion, enteral, rectal, or transdermal administration, inhalation, sublingual administration, or cutaneous, subcutaneous, intramuscular, intraocular, intraperitoneal, or intravenous injection, or any other route of administration known in the art for administrating therapeutic agents.

When the composition is to be administered orally, sublingually, or by any non-injectable route, the therapeutic formulation will preferably include a physiologically acceptable carrier, such as an inert diluent or an assimilable edible carrier with which the composition is administered. Suitable formulations that include pharmaceutically acceptable excipients for introducing compounds to the bloodstream by other than injection routes can be found in *Remington's Pharmaceutical Sciences* (18th ed.) (Genarro, ed. (1990) Mack Publishing Co., Easton, PA). The oligonucleotide and other ingredients may be enclosed in a hard or soft shell gelatin capsule, compressed into tablets, or incorporated directly into the individual's diet. The therapeutic compositions may be incorporated with excipients and used in the form of ingestible tablets, buccal tablets, troches, capsules, elixirs, suspensions, syrups, wafers, and the like. When the therapeutic composition is administered orally, it may be mixed with other food forms and pharmaceutically acceptable flavor enhancers. When the therapeutic composition is administered enterally, they may be introduced in a solid, semi-solid, suspension, or emulsion form and may be compounded with any number of well-known, pharmaceutically acceptable additives. Sustained release oral delivery systems and/or enteric coatings for orally administered dosage forms are also contemplated such as those described in U.S. Patent Nos. 4,704,295, 4,556,552, 4,309,404, and 4,309,406.

When a therapeutically effective amount of composition of the invention is administered by injection, the synthetic oligonucleotide will preferably be in the form of a pyrogen-free, parenterally-acceptable, aqueous solution. The preparation of such parenterally-acceptable solutions, having due regard to ph, isotonicity, stability, and the like, is within the skill in the art. A preferred pharmaceutical composition for injection should contain, in addition to the synthetic oligonucleotide, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

The pharmaceutical forms suitable for injectable use include sterile aqueous solutions or dispersions and sterile powders for the extemporaneous preparation of sterile injectable solutions or dispersions. In all cases the form must be sterile. It must be stable under the conditions of manufacture and storage and may be preserved against the contaminating action of microorganisms, such as bacterial and fungi. The carrier can be a solvent or dispersion medium. The prevention of the action of microorganisms can be brought about by various antibacterial and antifungal agents. Prolonged absorption of the injectable therapeutic agents can be brought about by the use of the compositions of agents delaying absorption. Sterile injectable solutions are prepared by incorporating the oligonucleotide in the required amount in the appropriate solvent, followed by filtered sterilization.

The pharmaceutical formulation can be administered in bolus, continuous, or intermittent dosages, or in a combination of continuous and intermittent dosages, as determined by the physician and the degree and/or stage of illness of the patient. The duration of therapy using the pharmaceutical composition of the present invention will vary, depending on the unique characteristics of the oligonucleotide and the particular therapeutic effect to be achieved, the limitations inherent in the art of preparing such a therapeutic formulation for the treatment of humans, the severity of the disease being treated and the condition and potential idiosyncratic response of each individual patient. Ultimately the attending physician will decide on the appropriate duration of intravenous therapy using the pharmaceutical composition of the present invention.

Compositions of the invention are useful for inhibiting or reducing the proliferation of cancer or tumor cells *in vitro.* A synthetic oligonucleotide of the invention is administered to the cells in an amount sufficient to enable the binding of the oligonucleotide to a complementary genomic region or RNA molecule transcribed therefrom encoding the RI_{α} subunit. In this way, expression of PKA is decreased, thus inhibiting or reducing cell proliferation.

Compositions of the invention are also useful for treating cancer or uncontrolled cell proliferation in humans. In this method, a therapeutic formulation including an antisense oligonucleotide of the invention is provided in a physiologically acceptable carrier. The individual is then treated with the therapeutic formulation in an amount sufficient to enable the binding of the oligonucleotide to the PKA RI_{α} genomic region or RNA molecule transcribed therefrom in the infected cells. In this way, the binding of the oligonucleotide inhibits or down-regulates RI_{α} expression and hence the activity of PKA.

In practicing the method of treatment or use of the present invention, a therapeutically effective amount of at least one or more therapeutic compositions of the invention is administered to a subject afflicted with a cancer. An anticancer response showing a decrease in tumor growth or size or a decrease in RI_{α} expression is considered to be a positive indication of the ability of the method and pharmaceutical formulation to inhibit or reduce cell growth and thus, to treat cancer in humans.

The composition of the invention is administered in accordance with the method of the invention in combination with paclitaxol.

### EXAMPLE 1

### Synthesis, Deprotection, and Purification of Oligonucleotides

Oligonucleotides were synthesized using standard phosphoramidite chemistry (Beaucage (1993) *Meth. Mol. Biol.* **20**:33-61) on an automated DNA synthesizer (Model 8700, Biosearch, Bedford, MA) using a beta-cyanoethyl phosphoramidate approach.

Oligonucleotide phosphorothioates were synthesized using an automated DNA synthesizer (Model 8700, Biosearch, Bedford, MA) using a beta-cyanoethyl phosphoramidate approach on a 10 micromole scale. To generate the phosphorothioate linkages, the intermediate phosphite linkage obtained after each coupling was oxidized using 3H, 1,2-benzodithiole-3H-one-1,1-dioxide (see Beaucage, in *Protocols for Oligonucleotides and Analogs: Synthesis and Properties,* Agrawal (ed.), (1993) Humana Press, Totowa, NJ, pp. 33-62). Similar synthesis was carried out to generate phosphodiester linkages, except that a standard oxidation was carried out using standard iodine reagent. Synthesis of inverted chimeric oligonucleotide was carried out in the same manner, except that methylphosphonate linkages were assembled using nucleoside methylphosphonamidite (Glen Research, Sterling, VA), followed by oxidation with 0.1 M iodine in tetrahydrofuran/2,6-lutidine/water (75:25:0.25) (see Agrawal & Goodchild (1987) *Tet. Lett.* 28:3539-3542). Hybrids and inverted hybrid oligonucleotides were synthesized similarly, except that the segment containing 2'-O-methylribonucleotides was assembled using 2'-O-methylribonucleoside phosphoramidite, followed by oxidation to a phosphorothioate or phosphodiester linkage as described above. Deprotection and purification of oligonucleotides was carried out according to standard procedures, (see Padmapriya et al. (1994) *Antisense Res. & Dev.* **4**:185-199), except for oligonucleotides containing methylphosphonate-containing regions. For those oligonucleotides, the CPG-bound oligonucleotide was treated with concentrated ammonium hydroxide for 1 hour at room temperature, and the supernatant was removed and evaporated to obtain a pale yellow residue, which was then treated with a mixture of ethylenediamine/ethanol (1:1 v/v) for 6 hours at room temperature and dried again under reduced pressure.

### EXAMPLE 2

### Propagation and Quantitation of Cell Lines and Virus Stocks

The cell line utilized was the CEM-SS cell line (Southern Research Institute-Frederick Research Center, Frederick, MD). These cells are highly susceptible to infection with HIV, rapidly form multinucleated syncytia, and are eventually killed by HIV. The cells were maintained (2-7 x 10⁵ cells per ml) in RPMI 1640 tissue culture medium supplemented with 10% fetal bovine serum, glutamine, and antibiotics, and were passaged twice weekly at 1:20 dilution. Passage number was logged each week. Cells were discarded after twenty weeks of passage and fresh CEM-SS cells thawed and utilized in the assay. Stocks of CEM-SS cells were frozen in liquid nitrogen in 1 ml NUNC vials in 90% fetal calf serum and 10% dimethyl sulfoxide (DMSO). Following thawing, CEM-SS cells were routinely ready to be utilized in the primary screen assay after two weeks in culture. Prior to replacing a late passage cell line, the new CEM-SS cells weree tested in the screening assay protocol utilizing the current stock of infectious virus and AZT. If the infectivity of the virus was significantly different on the new cells or if AZT appeared less active than expected the new cells were not entered into the screening program. Mycoplasma testing was routinely performed on all cell lines.

Virus utilized Southern Research Institute-Frederick Research Center. Virus pools were prepared and titrated in CEM-SS cells, placed in 5 ml aliquots, and frozen at -135°C. After thawing, unused virus is discarded to avoid changes in infectious titer. Virus pools were prepared by the acute infection of 5 x 10⁵ CEM-SS cells with HIV in a volume of 200 µl at a multiplicity of infection determined to give complete cell killing at day 7 post-infection (approximately 0.05 for the III_{B} isolate of HIV-1 and 0.01 for the RF isolate of HIV-1). Infection was allowed to proceed for one hour at 37°C, after which the cells were transferred to a T25 flask and the volume increased to 2 ml. On day 1 post-infection the volume was brought to 5 ml and on day 2 the volume was increased to 10 ml. Beginning on day 4, the cells were pelleted, the supernatant saved, and the cells resuspended in a fresh 10 ml aliquot of tissue culture medium. Complete medium changes on a daily basis, rather than allowing growth of the cells in the medium for longer periods of time, allowed the virus inoculum utilized in the primary screen to remain relatively undepleted of nutrients when it is used to infect cells. The staining reaction utilized (XTT, see method below) required that the glucose concentration remain high (161). Wells depleted of glucose by cell growth will not permit metabolic conversion of the tetrazolium dye to the formazan product.

Cell-free supernatants from the acutely infected cells were saved on day 4, day 5, day 6, and day 7. An aliquot of supernatant was saved separately on each day for use in titer determination. Titer determinations included reverse transcriptase activity assay (see below), endpoint titration or plaque assay (CEM-SS) quantification of infectious particles (see below), and quantification of cell killing kinetics.

It has been determined that peak levels of infectious virus are produced in the acutely infected cultures as the viability of the cells falls through the 50% level. Since the primary screening assay quantifies the protective effects of a compound by its ability to inhibit HIV-induced cytopathic effects, the quantity of virus required to kill CEM-SS cells in 6 days was routinely utilized to determine the amount of virus required per well in the primary screening assay. Each of the daily pools was titrated in the primary screening tetrazolium dye XTT assay protocol by performing two-fold dilutions of the virus beginning at a high test concentration of 50 µl of virus per well. The XTT staining method was utilized to determine the exact amount of virus required to kill all of the CEM-SS cells in each well and this minimum amount of virus was utilized for performance of all primary testing. Identical methods were utilized to prepare all virus isolates utilized, including laboratory-derived strains of HIV-1, HIV-2 and SIV. Clinical isolates utilized were passaged in fresh human cells. The methods for the growth of these cells and the production of virus pools is described below.

### Titer determinations

reverse transcriptase activity assay (see methods below), endpoint titration or plaque assay (CEM-SS) quantification of infectious particles (see methods below), and quantification of cell killing kinetics.

### Microtiter Antiviral XTT Assay

The tetrazolium dye XTT staining method was utilized to determine the exact amount of virus required to kill all of the CEM-SS cells in each well and this minimum amount of virus was utilized for performance of all primary testing.

### Cell Preparation:

CEM-SS cells (or other established human cell line used in these experiments) were passaged in T-150 flasks for use in the assay. On the day preceding the assay, the cells were split I:2 to assure they would be in an exponential growth phase at time of infection. On the day of assay the cells were washed twice with tissue culture medium and resuspended in fresh tissue culture medium. Total cell and viability counting was performed using a hemacytometer and trypan blue dye exclusion. Cell viability was greater than 95% for the cells to be utilized in the assay. The cells were pelleted and resuspended at 2.5 x 10⁴ cells per ml in tissue culture medium. Cells were added to the drug-containing plates in a volume of 50 µl.

### Virus Preparation:

A pretitered aliquot of virus was removed from the freezer -80°C) and allowed to thaw slowly to room temperature in a biological safety cabinet. The virus was resuspended and diluted into tissue culture medium such that the amount of virus added to each well in a volume of 50 µl will be the amount determined to give complete cell killing at 6 days post-infection. In general the virus pools produced with the IIIB isolate of HIV required the addition of 5 µl of virus per well. Pools of RF virus were five to ten-fold more potent, requiring 0.5-1 µl per well. TCID₅₀ calculation by endpoint titration in CEM-SS cells indicated that the multiplicity of infection of these assays ranged from 0.005-2.5.

### Plate Format:

Each plate contained cell control wells (cells only), virus control wells (cells plus virus), drug toxicity control wells (cells plus drug only), drug colorimetric control wells (drug only) as well as experimental wells (drug plus cells plus virus).

### XTT Staining of Screening Plates:

After 6 days of incubation at 37°C in a 5% CO₂ incubator the test plates were analyzed by staining with the tetrazolium dye XTT. XTT-tetrazolium is metabolized by the mitochondrial enzymes of metabolically active cells to a soluble formazan product, allowing the rapid quantitative analysis of the inhibition of HIV-induced cell killing by anti-HIV test substances. On day 6 post-infection plates were removed from the incubator and observed. The use of round bottom microtiter plates allows rapid macroscopic analysis of the activity of a given test compound by the evaluation of pellet size. The results of the macroscopic observations were confirmed and enhanced by further microscopic analysis.

XTT solution was prepared daily as a stock of 1 mg/ml in PBS. Phenazine methosulfate (PMS) solution was prepared at 15 mg/ml in PBS and stored in the dark at -20°C. XTT/PMS stock was prepared immediately before use by diluting the PMS 1:100 into PBS and adding 40 µl per ml of XTT solution. Fifty microliters of XTT/PMS was added to each well of the plate and the plate was incubated for an additional 4 hours at 37°C. Adhesive plate sealers were used in place of the lids, the sealed plate was inverted several times to mix the soluble formazan product and the plate was read spectrophotometrically at 450 nm with a Molecular Devices Vmax plate reader. Using an in-house computer program %CPE Reduction, %Cell Viability, IC₂₅, _{50 & 95}, TC_{25.50 & 95} and other indices were calculated and the graphic results summary was displayed.

### b. Reverse Transcriptase Activity Assay:

A microtiter based reverse transcriptase (RT) reaction was utilized (Buckheit *et al* (1991) *AIDS Research and Human Retroviruses* 7:295-302). Tritiated thymidine triphosphate (NEN) (TTP) was resuspended in distilled H₂O at 5 Ci/ml. Poly rA and oligo dT were prepared as a stock solution which was kept at -20°C. The RT reaction buffer was prepared fresh on a daily basis and consists of 125 µl 1M EGTA, 125 µl dH₂O, 125 µl Triton X-100, 50 µl 1M Tris(pH 7.4), 50 µl 1M DTT, and 40 µl 1M MgCl₂. These three solutions were mixed together in a ratio of 1 parts distilled water. Ten microliters of this reaction mixture was placed in a round bottom microtiter plate and 15 µl of virus containing supernatant was added and mixed. The plate was incubated at 37°C and incubated for 60 minutes. Following reaction, the reaction volume was spotted onto filter mats, washed 6 times for 5 minutes each in a 5% sodium phosphate buffer, 2 times for 1 minute each in distilled water, 2 times for 1 minute each in 70% ethanol, and then dried. The dried filter mat was placed in a plastic sample bag, Betaplate scintillation fluid was added and the bag was heat-sealed. Incorporated radioactivity was quantified utilizing a Wallac Microbeta scintillation counter.

### c. p24 ELISA:

ELISA kits were purchased from Coulter. The assay is performed according to the manufacturer's recommendations. Prior to ELISA analysis we routinely performed the reverse transcriptase activity assays (described above) and used the values for incorporated radioactivity in the RT activity assay to determine the dilution of our samples requires for the ELISA. We have constructed standard curves so that the dilutions of virus to be used in the p24 ELISA can be accurately determined from the RT activity assay. Control curves are generated in each assay to accurately quantify the amount of capsid protein in each sample. Data was obtained by spectrophotometric analysis at 450 nm using a Molecular Devices Vmax plate reader. P24 concentrations were calculated from the optical density values by use of the Molecular Devices software package Soft Max.

### d. Infectious Particles:

Infectious virus particles were qualified utilizing the CEM-SS plaque assay as described by Nara, P.L. and Fischinger, P.J. (1988) Quantitative infectivity assay for HIV-1 and HIV-2 *Nature* 332:469-470). Flat bottom 96-well microtiter plates (Costar) were coated with 50 µl of poly-L-lysine (Sigma) at 50 µg/ml for 2 hours at 37°C. The wells were then washed with PBS and 2.5 x 10⁵ CEM-SS cells were placed in the microtiter well where they became fixed to the bottom of the plate. Enough cells were added to form a monolayer of CEM-SS cells in each well. Virus containing supernatant was added from each well of the XTT plate, including virus and cell controls and each serial dilution of the test substance. The number of syncytia were qualified in the flat-bottom 96-well microtiter plate with an Olympus CK2 inverted microscope at 4 days following infection. Each syncytium resulted from a single infectious HIV virion.

### Anti-HIV Activity in Fresh Human Cells: Assay in Fresh Human T-Lymphocytes

Fresh human peripheral blood lymphocytes (PBL) are isolated from voluntary Red Cross donors, seronegative for HIV and HBV. Leukophoresed blood is diluted 1:1 with Dulbecco's phosphate buffered saline (PBS), layered over 14 mL of Ficoll-Hypaque density gradient in a 50 mL centrifuge tube. Tubes are then centrifuged for 30 minutes at 600 X g. Banded PBLs are gently aspirated from the resulting interface and subsequently washed 2X with PBS by low speed centrifugation. After final wash, cells are enumerated by trypan blue exclusion and re-suspended at 1 x 10⁷/mL in RPMI 1640 with 15% Fetal Bovine Serum (FBS), 2 mM L-glutamine, 4 mg/mL PHA-P and allowed to incubate for 48 - 72 hours at 37°C. After incubation, PBLs are centrifuged and reset in RPMI 1640 with 15% FBS, 2 mM L-glutamine, 100 U/mL penicillin, 100 µg/mL streptomycin, 10 µg/mL gentamycin, and 20 U/mL recombinant human IL-2. PBLs are maintained in this medium at a concentration of 1-2 x 10⁶/mL with bi-weekly medium changes, until use in assay protocol.

For the PBL assay, PHA-P stimulated cells from at least two normal donors are pooled, set in fresh medium at 2 x 10⁶/mL and plated in the interior wells of a 96 well round bottom microplate at 50 µL/well. Test drug dilutions are prepared at a 2X concentration in microtiter tubes and 100 µL of each concentration is placed in appropriate wells in a standard format. Fifty microliters of a predetermined dilution of virus stock is placed in each test well. Wells with cells and virus alone are used for virus control. Separate plates are identically set without virus for drug cytotoxicity studies using an XTT assay system.

In the standard PBL assay (MOI: 0.2), the assay was ended on day 7 following collection of cell free supernatant samples for reverse transcriptase activity assay. In the low MOI PBL assay (MOI: 0.02), supernatant samples were collected on day 6, day 11, and day 14 post-infection and analyzed for RT activity. Tritiated thymidine triphosphate (NEN) (TTP) was resuspended in distilled H₂O at 5 Ci/ml. Poly rA and oligo dT were prepared as a stock solution which was kept at -20°C. The RT reaction buffer was prepared fresh on a daily basis and consists of 125 µl 1M DTT, and 40 µl 1M MgCl₂. These three solutions were mixed together in a ratio of 2 parts TTP, 1 part poly rA:oligo dT, and 1 part reaction buffer. Ten microliters of this reaction mixture was placed in a round bottom microtiter plate and 15 µl of virus containing supernatant was added and mixed. The plate was incubated at 37°C in a water bath with a solid support to prevent submersion of the plate and incubated for 60 minutes. Following reaction, the reaction volume was spotted onto pieces of DE81 paper, washed 5 times for 5 minutes each in a 5% sodium phosphate buffer, 2 times for 1 minute each in distilled water, 2 times for 1 minute each in 70% ethanol, and then dried. Opti-Fluor O was added to each sample and incorporated radioactivity was quantified utilizing a Wallac 1450 Microbetaplus liquid scintillation counter. Tritiated thymidine incorporation was measured in parallel cultures at day 7. Each well was pulsed with 1 µCi of tritiated thymidine and the cells were harvested 18 hours later with a Skatron cell harvester onto glass fiber filter papers. The filters were dried, placed in a scintillation vial with 1 ml of scintillation cocktail and incorporated radioactivity was quantified on a Packard Tri-Carb 1900 TR liquid scintillation counter.

### Anti-HIV Activity in Fresh Human Cells: Assay in Fresh Human Monocyte-Macrophages

For isolation of adherent cells, 3 x 10⁶ non-PHA stimulated peripheral blood cells were resuspended in Hanks buffered saline (with calcium and magnesium) supplemented with 10% human AB serum. The cells were placed in a 24-well microtiter plate at 37°C for 2 hours. Non-adherent cells were removed by vigorously washing six times. The adherent cells were cultured for 7 days in RPMI 1640 tissue culture medium with 15% fetal bovine serum. The cultures were carefully monitored for confluency during this incubation period. Infection of the cells was performed with the monocytotropic HIV-1 strains BaL or ADA and the matched pair of AZT-sensitive and AZT-resistant virus isolates. Each of these virus isolates was obtained from the NIAID AIDS Research and Reference Reagent Program. High titer pools of each of these viruses have been harvested from infected cultures of peripheral blood adherent cells and frozen in 1.0 ml aliquots at -80°C. Monocyte-macrophage monolayers were infected at an MOI of 0.1. Compounds to be evaluated in the monocyte-macrophage assay are added to the monolayers shortly before infection in order to maximize the potential for identifying active compounds.

At 2 days post-infection, the medium was decanted and the cultures washed twice with complete medium in order to remove excess virus. Fresh medium alone or medium containing the appropriate concentrations of drugs was added and incubation continued for an additional 5 days. XTT-tetrazolium or trypan blue exclusion assays (for cell viability) and HIV p24 ELISA assays (for production of p24 core antigen) were performed on Day 7 post-infection. ELISA kits were purchased from Coulter. The assay is performed according to the manufacturer's recommendations. Control curves are generated in each assay to accurately quantify the amount of capsid protein in each sample. Data was obtained by spectrophotometric analysis at 450 nm using a Molecular Devices Vmax plate reader. P24 concentrations were calculated from the optical density values by use of the Molecular Device software package Soft Max.

To determine the relative effect of inverted hybrid or inverted chimeric structure on oligonucleotide-mediated depletion of complement, the following experiments were performed. Venous blood was collected from healthy adult human volunteers. Serum was prepared for hemolytic complement assay by collecting blood into vacutainers (Becton Dickinson #6430 Franklin Lakes, NJ) without commercial additives. Blood was allowed to clot at room temperature for 30 minutes, chilled on ice for 15 minutes, then centrifuged at 4°C to separate serum. Harvested serum was kept on ice for same day assay or, alternatively, stored at -70°C. Buffer, or an oligonucleotide sample was then incubated with the serum. The oligonucleotides tested were 25mer oligonucleotide phosphodiesters or phosphorothioates, 25mer hybrid oligonucleotides, 25mer inverted hybrid oligonucleotides, 25mer chimeric oligonucleotides, and 25mer inverted chimeric oligonucleotides. Hybrid oligonucleotides were composed of seven to 13 2-O-methyl ribonucleotides flanked by two regions of six to nine deoxyribonucleotides each. 25mer inverted hybrid oligonucleotides were composed of 17 deoxyribonucleotides flanked by two regions of four ribonucleotides each. 25mer chimeric oligonucleotides were composed of six methylphosphonate deoxyribonucleotides and 19 phosphorothioate deoxyribonucleotides Inverted chimeric oligonucleotides were composed of from 16 to 17 phosphorothioate deoxyribonucleotides flanked by regions of from two to seven methylphosphonate deoxyribonucleotides, or from six to eight methylphosphonate deoxyribonucleotides flanked by nine to ten phosphorothioate deoxyribonucleotides, or two phosphorothioate regions ranging from two to 12 oligonucleotides, flanked by three phosphorothioate regions ranging in size from two to six nucleotides in length. A standard CH50 assay (See Kabat and Mayer (eds), *Experimental Immunochemistry,* 2d Ed., Springfield, IL, CC Thomas, p. 125) for complement-mediated lysis of sheep red blood cells (Colorado Serum Co.) sensitized with anti-sheep red blood cell antibody (hemolysin, Diamedix, Miami, FL) was performed, using duplicate determinations of at least five dilutions of each test serum, then hemoglobin release into cell-free supernates was measured spectrophotometrically at 541 nm.

### EXAMPLE 3

### In Vitro Mitogenicity Studies

To determine the relative effect of inverted hybrid or inverted chimeric structure on oligonucleotide-mediated mitogenicity, the following experiments were performed. Spleen was taken from a male CD1 mouse (4-5 weeks, 20-22 g; Charles River, Wilmington, MA). Single cell suspensions were prepared by gently mincing with frosted edges of glass slides. Cells were then cultured in RPMI complete media (RPMI media supplemented with 10% fetal bovine serum (FBS), 50 micromolar 2-mercaptoethanol (2-ME), 100 U/ml penicillin, 100 micrograms/ml streptomycin, 2 mM L-glutamine). To minimize oligonucleotide degradation, FBS was first heated for 30 minutes at 65°C (phosphodiester-containing oligonucleotides) or 56°C (all other oligonucleotides). Cells were plated in 96 well dishes at 100,000 cells per well (volume of 100 microliters/well). One type of each oligonucleotide described in Example 2 above in 10 microliters TE buffer (10 mM Tris-HCl, pH 7.5, 1 mM EDTA) was added to each well. After 44 hours of culturing at 37°C, one microcurie tritiated thymidine (Amersham, Arlington Heights, IL) was added in 20 microliters RPMI media for a 4 hour pulse labelling. The cells were then harvested in an automatic cell harvester (Skatron, Sterling, VA) and the filters were assessed using a scintillation counter. In control experiments for mitogenicity, cells were treated identically, except that either media (negative control) or concanavalin A (positive control) was added to the cells in place of the oligonucleotides.

All of the inverted hybrid oligonucleotides proved to be less immunogenic than phosphorothioate oligonucleotides. Inverted hybrid oligonucleotides having phosphodiester linkages in the 2'-O-methyl region appeared to be slightly less immunogenic than those containing phosphorothioate linkages in that region. No significant difference in mitogenicity was observed when the 2'-O-methyl ribonucleotide region was pared down from 13 to 11 or to 9 nucleotides. Inverted chimeric oligonucleotides were also generally less mitogenic than phosphorothioate oligonucleotides. In addition, these oligonucleotides appeared to be less mitogenic than traditional chimeric oligonucleotides, at least in cases in which the traditional chimeric oligonucleotides had significant numbers of methylphosphonate linkages near the 3' end. Increasing the number of methylphosphonate linkers in the middle of the oligonucleotide from 5 to 6 or 7 did not appear to have a significant effect on mitogenicity. These results indicate that incorporation of inverted hybrid or inverted chimeric structure into an oligonucleotide can reduce its mitogenicity.

### EXAMPLE 4

### In Vitro Studies

To determine the relative effect of inverted hybrid or inverted chimeric structure on oligonucleotide-induced mitogenicity, the following experiments were performed. Venous blood was collected from healthy adult human volunteers. Plasma for clotting time assay was prepared by collecting blood into siliconized vacutainers with sodium citrate (Becton Dickinson #367705), followed by two centrifugations at 4°C to prepare platelet-poor plasma. Plasma aliquots were kept on ice, spiked with various test oligonucleotides described in Example 2 above, and either tested immediately or quickly frozen on dry ice for subsequent storage at -20°C prior to coagulation assay. Activated partial thromboplastin time (aPTT) was performed in duplicate on an Electra 1000C (Medical Laboratory Automation, Mount Vernon, NY) according to the manufacturer's recommended procedures, using Actin FSL (Baxter Dade, Miami, FL) and calcium to initiate clot formation, which was measured photometrically. Prolongation of aPTT was taken as an indication of clotting inhibition side effect produced by the oligonucleotide.

Traditional phosphorothioate oligonucleotides produced the greatest prolongation of aPTT, of all of the oligonucleotides tested. Traditional hybrid oligonucleotides produced somewhat reduced prolongation of aPTT. In comparison with traditional phosphorothioate or traditional hybrid oligonucleotides, all of the inverted hybrid oligonucleotides tested produced significantly reduced prolongation of aPTT. Inverted hybrid oligonucleotides having phosphodiester linkages in the 2'-O-substituted ribonucleotide region had the greatest reduction in this side effect, with one such oligonucleotide having a 2'-O-methyl RNA phosphodiester region of 13 nucleotides showing very little prolongation of aPTT, even at oligonucleotide concentrations as high as 100 micrograms/ml. Traditional chimeric oligonucleotides produce much less prolongation of aPTT than do traditional phosphorothioate oligonucleotides. Generally, inverted chimeric oligonucleotides retain this characteristic. At least one inverted chimeric oligonucleotide, having a methylphosphonate region of seven nucleotides flanked by phosphorothioate regions of nine nucleotides, gave better results in this assay than the traditional chimeric oligonucleotides at all but the highest oligonucleotide concentrations tested. These results indicate that inverted hybrid and inverted chimeric oligonucleotides may provide advantages in reducing the side effect of clotting inhibition when they are administered to modulate gene expression *in vivo .*

### EXAMPLE 5

### In Vivo Complement Activation Studies

Rhesus monkeys (4-9 kg body weight) are acclimatized to laboratory conditions for at least 7 days prior to the study. On the day of the study, each animal is lightly sedated with ketamine-HCl (10 mg/kg) and diazepam (0.5 mg/kg). Surgical level anesthesia is induced and maintained by continuous ketamine intravenous drip throughout the procedure. The oligonucleotides described in Example 2 above are dissolved in normal saline and infused intravenously via a cephalic vein catheter, using a programmable infusion pump at a delivery rate of 0.42 mg/minute. For each oligonucleotide, doses of 0, 0.5, 1, 2, 5 and 10 mg/kg are administered to two animals each over a 10 minute infusion period. Arterial blood samples are collected 10 minutes prior to oligonucleotide administration and 2, 5, 10, 20, 40 and 60 minutes after the start of the infusion, as well as 24 hours later. Serum is used for determining complement CH50, using the conventional complement-dependent lysis of sheep erythrocyte procedure (see Kabat and Mayer, 1961, *supra).* At the highest dose, phosphorothioate oligonucleotide causes a decrease in serum complement CH50 beginning within 5 minutes of the start of infusion. Inverted hybrid and chimeric oligonucleotides are expected to show a much reduced or undetectable decrease in serum complement CH50 under these conditions.

### EXAMPLE 6

### In Vivo mitogenicity Studies

CD1 mice are injected intraperitoneally with a dose of 50 mg/kg body weight of oligonucleotide described in Example 2 above. Forty-eight hours later, the animals are euthanized and the spleens are removed and weighed. Animals treated with inverted hybrid or inverted hybrid oligonucleotides are expected to show no significant increase in spleen weight, while those treated with oligonucleotide phosphorothioates are expected to show modest increases in spleen weight.

### EXAMPLE 7

### In Vivo Clotting Studies

Rhesus monkeys are treated as in Example 5. From the whole blood samples taken, plasma for clotting assay is prepared, and the assay performed, as described in Example 4. It is expected that prolongation of aPTT will be substantially reduced for both inverted hybrid oligonucleotides and for inverted chimeric oligonucleotide, relative to traditional oligonucleotide phosphorothioates.

### EXAMPLE 8

### RNase H Activity Studies

To determine the ability of inverted hybrid oligonucleotides and inverted chimeric oligonucleotides to activate RNase H when bound to a complementary RNA molecule, the following experiments were performed. Each type of oligonucleotide described in Example 2 above was incubated together with a molar equivalent quantity of complimentary oligoribonucleotide (0.266 micromolar concentration of each), in a cuvette containing a final volume of 1 ml RNase H buffer (20 mM Tris-HCl, pH 7.5, 10 mM MgCl₂, 0.1 M KCl, 2% glycerol, 0.1 mM DTT). The samples were heated to 95°C, then cooled gradually to room temperature to allow annealing to form duplexes. Annealed duplexes were incubated for 10 minutes at 37°C, then 5 units RNase H was added and data collection commenced over a three hour period. Data was collected using a spectrophotometer (GBC 920, GBC Scientific Equipment, Victoria, Australia) at 259 nm. RNase H degradation was determined by hyperchromic shift.

As expected, phosphodiester oligonucleotides behaved as very good co-substrates for RNase H-mediated degradation of RNA, with a degradative half-life of 8.8 seconds. Phosphorothioate oligonucleotides produced an increased half-life of 22.4 seconds. Introduction of a 2'-O-methyl ribonucleotide segment at either end of the oligonucleotide further worsened RNase H activity (half-life = 32.7 seconds). In contrast, introducing a 2'-O-methyl segment into the middle of the oligonucleotide (inverted hybrid structure) always resulted in improved RNase H-mediated degradation. When a region of 13 2'-O-methylribonucleoside phosphodiesters was flanked on both sides by phosphorothioate DNA, the best RNase H activity was observed, with a half-life of 7.9 seconds. Introduction of large blocks of methylphosphonate-linked nucleosides at the 3' end of the oligonucleotide either had no effect or caused further deterioration of RNase H activity even when in a chimeric configuration. Introduction of methylphosphonate linked nucleosides at the 5' end, however, improved RNase H activity, particularly in a chimeric configuration with a single methylphosphonate linker at the 3' end (best half-life = 8.1 seconds). All inverted chimeric oligonucleotides with methylphosphonate core regions flanked by phosphorothioate regions gave good RNase results, with a half-life range of 9.3 to 14.4 seconds. These results indicate that the introduction of inverted hybrid or inverted chimeric structure into phosphorothioate-containing oligonucleotides can restore some or all of the ability of the oligonucleotide to act as a co-substrate for RNase H, a potentially important attribute for an effective antisense agent.

### EXAMPLE 9

### Melting Temperature Studies

To determine the effect of inverted hybrid or inverted chimeric structure on stability of the duplex formed between an antisense oligonucleotide and a target molecule, the following experiments were performed. Thermal melting (Tm) data were collected using a spectrophotometer (GBC 920, GBC Scientific Equipment, Victoria, Australia), which has six 10 mm cuvettes mounted in a dual carousel. In the Tm experiments, the temperature was directed and controlled through a peltier effect temperature controller by a computer, using software provided by GBC, according to the manufacturer's directions. Tm data were analyzed by both the first derivative method and the midpoint method, as performed by the software. Tm experiments were performed in a buffer containing 10 mM PIPES, pH 7.0, 1 mM EDTA, 1 M NaCl. A refrigerated bath (VWR 1166, VWR, Boston, MA) was connected to the peltier-effect temperature controller to absorb the heat. Oligonucleotide strand concentration was determined using absorbance values at 260 nm, taking into account extinction coefficients.

### EXAMPLE 10

### Tumor Growth and Antisense Treatment

LS-174T human colon carcinoma cells (1 x 10⁶ cells) were inoculated subcutaneously (s.c.) into the left flank of athymic mice. A single dose of RI_{α} antisense hybrid (Oligo 165 SEQ ID NO:4), inverted hybrid (Oligo 166, SEQ ID NO:6), or inverted chimeric (Oligo 190, SEQ ID NO:1) oligonucleotides or control oligonucleotide (Oligo 169, SEQ ID NO:7); oligo 168 (SEQ ID NO:5); oligo 188, SEQ ID NO:3)) as shown in Table 1 (1 mg per 0.1 ml saline per mouse), or saline (0.1 ml per mouse), was injected s.c. into the right flank of mice when tumor size reached 80 to 100 mg, about 1 week after cell inoculation. Tumor volumes were obtained from daily measurement of the longest and shortest diameters and calculation by the formula, 4/3πr³ where r = (length + width)/4. At each indicated time, two animals from the control and antisense-treated groups were killed, and tumors were removed and weighed. The results are shown in FIG. 1. These results show that the size of the tumor in the animal treated with the inverted hybrid oligonucleotide 166 having SEQ ID NO:6 was surprisingly smaller from three days after injection onward than the phosphorothioate oligonucleotide 164 having SEQ ID NO:1. That this effect was sequence-specific is also demonstrated in FIG. 1: control oligonucleotide 168 (SEQ ID NO:3) has little ability to keep tumor size at a minimum relative to the hybrid and inverted hybrid oligonucleotides.

### EXAMPLE 11

### Photoaffinity Labelling and Immunoprecipitation of RI_{α} Subunits

The tumors are homogenized with a Teflon/glass homogenizer in ice-cold buffer 10 (Tris-HCl, pH 7.4, 20 mM; NaCl, 100 mM; NP-40, 1%; sodium deoxycholate, 0.5%; MgCl₂, 5 mM; pepstatin, 0.1 mM; antipain, 0.1 mM; chymostatin, 0.1 mM; leupeptin, 0.2 mM; aprotinin, 0.4 mg/ml; and soybean trypsin inhibitor, 0.5 mg/ml; filtered through a 0.45-µm pore size membrane), and centrifuged for 5 min in an Eppendorf microfuge at 4°C. The supernatants are used as tumor extracts.

The amount of PKA RI_{α} subunits in tumors is determined by photoaffinity labelling with 8-N₃-[³²P]cAMP followed by immunoprecipitation with RI_{α} antibodies as described by Tortora et al. *(Proc. Natl. Acad. Sci. (USA)* (1990) **87**:705-708). The photoactivated incorporation of 8-N₃-[³²P]cAMP (60.0 Ci/m-mol), and the immunoprecipitation using the anti-RI_{α} or anti-RII_{β} antiserum and protein A Sepharose and SDS-PAGE of solubilized antigen-antibody complex follows the method previously described (Tortora et al. (1990) *Proc. Natl. Acad Sci. (USA)* 87:705-708; Ekanger et al. (1985) *J. Biol. Chem.* **260**:3393-3401). It is expected that the amount of RI_{α} in tumors treated with hybrid, inverted hybrid, and inverted chimeric oligonucleotides of the invention will be reduced compared with the amount in tumors treated with mismatch, straight phosphorothioate, or straight phosphodiester oligonucleotide controls, saline, or other controls.

### EXAMPLE 12

### cAMP-Dependent Protein Kinase Assays

Extracts (10 mg protein) of tumors from antisense-, control antisense-, or saline-treated animals are loaded onto DEAE cellulose columns (1 x 10 cm) and fractionated with a linear salt gradient (Rohlff et al. (1993) *J. Biol. Chem.* **268**:5774-5782). PKA activity is determined in the absence or presence of 5 µM cAMP as described below (Rohlff et al . (1993) *J. Biol. Chem.* **268**:5774-5782). cAMP-binding activity is measured by the method described previously and expressed as the specific binding (Tagliaferri et al. (1988) *J. Biol. Chem.* **263**:409-416).

After two washes with Dulbecco's phosphate-buffered saline, cell pellets (2 x 10⁶ cells) are lysed in 0.5 ml of 20 mM Tris (pH 7.5), 0.1 mM sodium EDTA, 1 mM dithiothreitol, 0.1 mM pepstatin, 0.1 mM antipain, 0.1 mM chymostatin, 0.2 mM leupeptin, 0.4 mg/ml aprotinin, and 0.5 mg/ml soybean trypsin inhibitor, using 100 strokes of a Dounce homogenizer. After centrifugation (Eppendorf 5412) for 5 min, the supernatants are adjusted to 0.7 mg protein/ml and assayed (Uhler et al. (1987) *J. Biol. Chem.* **262**:15202-15207) immediately. Assays (40 µl total volume) are performed for 10 min at 300°C and contained 200 µM ATP, 2.7 x 10⁶ cpm γ [³²P]ATP, 20 mM MgCl₂, 100 µM Kemptide (Sigma K-1127) (Kemp et al. (1977) *J. Biol. Chem.* 252:4888-4894), 40 mM Tris (pH 7.5), ± 100 µM protein kinase inhibitor (Sigma P-3294) (Cheng et al. (1985) *Biochem. J.* 231:655-661), ± 8 µM cAMP and 7 µg of cell extract. The phosphorylation of Kemptide is determined by spotting 20 µl of incubation mixture on phosphocellulose filters (Whatman, P81) and washing in phosphoric acid as described (Roskoski (1983) *Methods Enzymol.* **99**:3-6). Radioactivity is measured by liquid scintillation using Econofluor-2 (NEN Research Products NEF-969). It is expected that PKA and cAMP binding activity will be reduced in extracts of tumors treated with the hybrid, inverted hybrid, and inverted chimeric oligonucleotides.

## Claims

1. The use of a synthetic, modified oligonucleotide complementary to, and capable of down-regulating the expression of, nucleic acid encoding protein kinase A subunit RIa, the modified oligonucleotide having from about 15 to about 30 nucleotides and being a hybrid oligonucleotide comprising a region of at least two deoxyribonucleotides, flanked by 3' and 5' flanking ribonucleotide, flanked by 3' and 5' flanking ribonucleotide regions each having a least four ribonucleotides,
in combination with paclitaxol
for the preparation of a therapeutic composition for the treatment of cancer in an afflicted subject, wherein the oligonucleotide has substantially the nucleotide sequence set forth in SEQ ID No.:4.

2. The use of the oligonucleotide of claim 1, wherein the oligonucleotide has 18 nucleotides.

3. The use of the oligonucleotide of claim 1 wherein each of the flanking ribonucleotide regions of the oligonucleotide comprises at least four contiguous 2'-O-substituted ribonucleotides.

4. The use of the oligonucleotide of claim 3 wherein each of the flanking ribonucleotide regions of the oligonucleotide comprises at least one 2'-O-alkyl ribonucleotide.

5. The use of the claim 4 wherein each of the flanking ribonucleotide regions of the oligonucleotide comprises at least one 2'-O-methyl ribonucleotide.

6. The use of the oligonucleotide of claim 3 wherein each of the flanking ribonucleotide regions of the oligonucleotide comprises at least four 2'-O-methyl ribonucleotides.

7. The use of the oligonucleotide of claim 1 wherein the ribonucleotides and deoxyribonucleotides of the oligonucleotide are linked by phosphorothioate internucleotide linkages.

8. The use of claim 1, wherein the therapeutic composition is comprised in a pharmaceutically acceptable carrier.

## Patentansprüche

1. Verwendung eines synthetischen modifizierten Oligonukleotids komplementär zu, und fahig zur Herunterregulierung der Expression von, einer Nukleinsäure kodierend für eine Protein Kinase A Untereinheit RIa, wobei das modifizierte Oligonukleotid von ungefähr 15 bis ungefähr 30 Nukleotide besitzt und ein Hybrid Oligonukleotid ist, umfassend einen Bereich von wenigstens zwei Deoxyribonukleotiden, flankiert durch 3' und 5' flankierende Ribonukleotide, flankiert durch 3' und 5' flankierende Ribonukleotid-Regionen, jede besitzend wenigstens vier Ribonukleotide,
in Kombination mit Paclitaxol
für die Herstellung einer therapeutischen Zusammensetzung für die Behandlung von Krebs in einem betroffenen Subjekt, wobei das Oligonukleotid im Wesentlichen die Nukleotidsequenz besitzt, die in SEQ ID No.: 4 aufgeführt ist.

2. Verwendung des Oligonukleotids von Anspruch 1, wobei das Oligonukleotid 18 Nukleotide besitzt.

3. Verwendung des Oligonukleotids von Anspruch 1, wobei jede der flankierenden Ribonukleotid-Regionen des Oligonukleotids wenigsten vier aufeinanderfolgende 2'-O-substituierte Ribonukleotide umfasst.

4. Verwendung des Oligonukleotids von Anspruch 3, wobei jede der flankierenden Ribonukleotid-Regionen des Oligonukleotids wenigstens ein 2'-O-Alkyl-Ribonukleotid umfasst.

5. Verwendung des Anspruchs 4, wobei jede der flankierenden Ribonukleotid-Regionen des Oligonukleotids wenigstens ein 2'-O-Methyl-Ribonukleotid umfasst.

6. Verwendung des Oligonukleotids von Anspruch 3, wobei jede der flankierenden Ribonukleotid-Regionen des Oligonukleotids wenigstens vier 2'-O-Methyl-Ribonukleotide umfasst.

7. Verwendung des Oligonukleotids von Anspruch 1, wobei die Ribonukleotide und Deoxyribonukleotide des Oligonukleotids durch Phosphorothioat Intemukleotid-Bindungen verbunden sind.

8. Die Verwendung von Anspruch 1, wobei die therapeutische Zusammensetzung einen pharmazeutisch verträglichen Träger umfasst.

## Revendications

1. Utilisation d'un oligonucléotide de synthèse modifié, complémentaire de, et capable d'inhiber l'expression d'un acide nucléique codant pour la sous-unité RIa de la protéine kinase A, l'oligonucléotide modifié ayant environ 15 à environ 30 nucléotides et étant un oligonucléotide hybride comprenant une région d'au moins deux désoxy-ribonucléotides, flanquée d'un ribonucléotide adjacent en 3' et en 5', flanqué de régions ribonucléotidiques adjacentes en 3' et en 5', chacune ayant au moins quatre ribonucléotides, en combinaison avec du paclitaxol pour la préparation d'une composition thérapeutique pour le traitement d'un cancer chez un sujet affecté, dans laquelle l'oligonucléotide a sensiblement la séquence nucléotidique présentée en SEQ ID NO : 4.

2. Utilisation de l'oligonucléotide de la revendication 1, dans laquelle l'oligonucléotide a 18 nucléotides.

3. Utilisation de l'oligonucléotide de la revendication 1 dans laquelle chacune des régions ribonucléotidiques adjacentes de l'oligonucléotide comprend au moins quatre ribonucléotides contigus 2'-O-substitués.

4. Utilisation de l'oligonucléotide de la revendication 3 dans laquelle chacune des régions ribonucléotidiques adjacentes de l'oligonucléotide comprend au moins un ribonucléotide 2'-O-alkyle.

5. Utilisation de la revendication 4 dans laquelle chacune des régions ribonucléotidiques adjacentes de l'oligonucléotide comprend au moins un ribonucléotide 2'-O-méthyle.

6. Utilisation de l'oligonucléotide de la revendication 3 dans laquelle chacune des régions ribonucléotidiques adjacentes de l'oligonucléotide comprend au moins quatre ribonucléotides 2'-O-méthyle.

7. Utilisation de l'oligonucléotide de la revendication 1 dans laquelle les ribonucléotides et désoxyribonucléotides de l'oligonucléotide sont liés par des liaisons internucléotidiques phosphorothioate.

8. Utilisation de la revendication 1, dans laquelle la composition thérapeutique est comprise dans un véhicule pharmaceutiquement acceptable.


### SEQUENCE LISTING

(1) GENERAL INFORMATION:
   (i) APPLICANT: IDERA PHARMACEUTICAS, INC.
   (ii) TITLE OF INVENTION: MODIFIED PROTEIN KINASE A-SPECIFIC OLIGONUCLEOTIDE AND METHODS OF THEIR USE
   (iii) NUMBER OF SEQUENCES: 8
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (v) CURRENT APPLICATION DATA:
      (A) APPLICATION NUMBER: EP 98 907 490.1
      (B) FILING DATE: 12-FEB-1998
      (C) CLASSIFICATION:
(2) INFORMATION FOR SEQ ID NO:1:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:1:
      GCGTGCCTCC TCACTGGC 18
(2) INFORMATION FOR SEQ ID NO:2:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:2:
      GCGCGCCTCC TCGCTGGC 18
(2) INFORMATION FOR SEQ ID NO:3:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: YES
   (xi) SEQUENCE DESCRIPTION: SEQ ID NO:3:
      GCATGCTTCC ACACAGGC 18
(2) INFORMATION FOR SEQ ID NO:4:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:4:
      GCGUGCCTCC TCACUGGC 18
(2) INFORMATION FOR SEQ ID NO:5:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:5:
      GCGCGCCTCC TCGCUGGC 18
(2) INFORMATION FOR SEQ ID NO:6:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:6:
      GCGTGCCUCC UCACTGGC 18
(2) INFORMATION FOR SEQ ID NO:7:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:7:
      GCGCGCCUCC UCGCTGGC 18
(2) INFORMATION FOR SEQ ID NO:8:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA/RNA
   (iii) HYPOTHETICAL: NO
   (iv) ANTI-SENSE: YES
   (xi) SEQUENCE DESCRIPTION: SEQ ID NO:8:
      GCATGCAUCC GCACAGGC 18